# EUROPEAN PATENT SPECIFICATION

(11) **EP 4 384 099 B1**
(45) Date of publication and mention of the grant of the patent: **24.06.2026**
(21) Application number: 22856505.7
(22) Date of filing: 09.08.2022
(51) Int. Cl.: A61B 18/02, A61B 18/14, A61B 17/11, A61B 17/12, A61B 17/00, A61B 18/00, A61B 90/00

(54) **APPARATUS AND SYSTEM FOR CREATING CHRONICALLY STABLE ATRIAL SHUNT**
VORRICHTUNG UND SYSTEM ZUR ERZEUGUNG EINES CHRONISCH STABILEN VORHOF-SHUNTS
APPAREIL ET SYSTÈME POUR CRÉER UN SHUNT ATRIAL À STABILITÉ CHRONIQUE

(30) Priority: 11.08.2021 US 202117399344
(43) Date of publication of application: 19.06.2024
(73) Proprietor: Medtronic, Inc., Minneapolis, Minnesota 55432 (US)
(72) Inventor: YANG, Zhongping C., Minneapolis, Minnesota 55432 (US); COULOMBE, Nicolas, Minneapolis, Minnesota 55432 (US); RORVICK, Anthony W., Minneapolis, Minnesota 55432 (US); PEDERSON, Brian D., Minneapolis, Minnesota 55432 (US); SCHULHAUSER, Randal, Minneapolis, Minnesota 55432 (US); ANDERSON, David A., Minneapolis, Minnesota 55432 (US); BRANNAN, Joseph D., Minneapolis, Minnesota 55432 (US); STEWART, Mark T., Minneapolis, Minnesota 55432 (US); LALONDE, Jean-Pierre, Minneapolis, Minnesota 55432 (US)
(74) Representative: Zimmermann & Partner Patentanwälte mbB
(86) International application number: PCT/US2022/039836
(87) International publication number: WO 2023/018720

(56) References cited:
- US-A1- 2014 012 363
- US-A1- 2014 142 559
- US-A1- 2014 343 348
- US-A1- 2019 269 392
- US-A1- 2021 007 790

## Description

### FIELD

The present technology is generally related to devices and methods for creating an atrial shunt. The methods are not claimed but may be performed using claimed devices.

### BACKGROUND

Atrial shunting is a procedure used to treat certain cardiac defects and heart failure. During the procedure, a blood flow pathway is created between the right atrium and the left atrium such that blood flows between them. In a typical procedure, the septal wall separating the atria is cut with a puncturing device and a mechanical device such as a stent is left in place to prevent tissue regrowth and to maintain the shunt. However, such procedures may result in tissue regrowth thus reducing the effectiveness of the shunt.

In other procedure, the tissue surrounding the septal wall may be ablated with thermal energy, such as cryogenic energy to prevent tissue regrowth and to maintain the shunt. However, current prior art devices fail to isolate the portion of the septal wall being ablated from blood flowing within the left atrium and/or the right atrium. Thus, the blood warms the tissue being ablated with cryogenic energy and the atrial shunt begins to close reducing the effectiveness of the shunt procedure.
US 2021/007790 A1 relates to a medical device and treatment method. US 2014/343348 A1 relates to methods and devices for diagnosing, monitoring, or treating medical conditions through an opening through an airway wall.

### SUMMARY

The techniques of this disclosure generally relate devices and methods for creating and atrial shunt. The methods are not claimed but may be performed using claimed devices.

In one aspect, a non-claimed method of creating a shunt between a right atrium and a left atrium of a mammalian heart including puncturing an atrial septum between the right atrium and the left atrium to create a shunt. An ablation device having balloon is advanced at least partially through the shunt. The balloon is inflated and configured to thermally isolate the atrial septum from blood within the left atrium and the right atrium. Ablation energy is delivered to ablate the atrial septum.

In another aspect of this embodiment, the balloon is inflated before delivering ablation energy to the atrial septum.

In another aspect of this embodiment, delivering ablation energy to ablate the atrial septum includes delivering refrigerant to the balloon.

In another aspect of this embodiment, delivering refrigerant to the balloon includes spraying refrigerant to a middle portion of the balloon.

In another aspect of this embodiment, the balloon includes a pair of longitudinally spaced lobes with the middle portion disposed therebetween, each of the pair of lobes defines a first diameter and the middle portion defines a second diameter less than the first diameter.

In another aspect of this embodiment, the atrial septum has a first side and a second side, and wherein inflating the balloon further includes inflating the first and second lobes of the pair of lobes to abut each of the first side and the second side, respectively.

In another aspect of this embodiment, the ablation device includes a first plurality of spray ports, and wherein the first plurality of spray ports is disposed proximate the middle portion of the balloon.

In another aspect of this embodiment, the ablation device defines a major longitudinal axis and wherein the first plurality of spray ports is angled in a direction orthogonal to the major longitudinal axis.

In another aspect of this embodiment, the first plurality of spray ports is included on a first coiled fluid injection tube, and wherein the ablation device further includes a second coiled fluid injection tube having a second plurality of spray ports, the second plurality of spray ports is angled in a direction orthogonal to the major longitudinal axis.

In another aspect of this embodiment, delivering ablation energy to ablate the atrial septum include delivering radiofrequency energy.

In one aspect, a medical device includes an elongate body defining a major longitudinal axis and having a proximal portion and a distal portion. The distal portion includes a balloon, the balloon includes a pair of longitudinally spaced lobes having a first diameter and a middle portion having a second diameter less than the first diameter disposed therebetween. An ablation element is disposed substantially within the middle portion, the ablation element is configured to deliver ablation energy to the middle portion.

In another aspect of this embodiment, the ablation element includes a first plurality of spray ports configured to deliver refrigerant to the middle portion.

In another aspect of this embodiment, the first plurality of spray ports is angled in a direction orthogonal to the major longitudinal axis.

In another aspect of this embodiment, the device further includes a second plurality of spray ports within the middle portion and longitudinally spaced from the first plurality of spray ports.

In another aspect of this embodiment, the ablation element is configured to deliver radiofrequency ablation energy.

In another aspect of this embodiment, the first lobe and the second lobe are sized and configured to, when inflated, abut and thermally isolate an atrial septum from blood flowing within a left atrium and a right atrium when the balloon is disposed within an atrial shunt.

In one aspect, a non-claimed method of creating a shunt between a right atrium and a left atrium of a mammalian heart, the method includes puncturing an atrial septum between the right atrium and the left atrium to create a shunt. A medical device having a balloon at least partially through the shunt. Pulse field ablation energy is delivered from the medical device to ablate the atrial septum.

In another aspect of this embodiment, the atrial septum has a first side and a second side opposite the first side, and wherein advancing the medical device having the balloon includes advancing the balloon entirely through the shunt and inflating the balloon to abut the second side of the atrial septum.

In another aspect of this embodiment, delivering pulse field ablation energy from the medical device includes advancing a plurality of electrodes to a position to abut the first side of the atrial septum.

In another aspect of this embodiment, the plurality of electrodes is configured to be disposed in a planar configuration adjacent the first side of the atrial septum.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is a system view of an exemplary medical device and system for creating an atrial shunt and constructed in accordance with the principles of the present application;
FIG. 2 is a side cross-sectional view of the distal portion of the medical device shown in FIG. 1;
FIG. 3 is a side cross-sectional view of another embodiment of the distal portion of the medical device shown in FIG. 1;
FIG. 4 is a system view of another exemplary medical device and system for creating an atrial shunt and constructed in accordance with the principles of the present application;
FIG. 5 is a side cross-sectional view of the distal portion of the medical device shown in FIG. 4;
FIG. 6 is a system view of another exemplary medical device and system for creating an atrial shunt and constructed in accordance with the principles of the present application;
FIG. 7 is a side view of the distal portion of the medical device shown in FIG. 6;
FIG. 8 is a step-by-step side view of an exemplary procedure for creating an atrial shunt with cryo-ablation energy;
FIG. 9 is a side view of the medical device shown in FIG. 1 creating an atrial shunt and thermally isolating septal tissue; and
FIG. 10 is a step-by-step side view of an exemplary procedure for creating an atrial shunt with pulse field ablation energy;
FIG. 11 is a system view of another exemplary medical device and system for creating an atrial shunt and constructed in accordance with the principles of the present application;
FIG. 12 is a step-by-step side view of another exemplary procedure for creating an atrial shunt with cryo-ablation energy; and
FIG. 13 is a side view of the medical device shown in FIG. 11 treating septal tissue with a therapeutic drug and creating an atrial shunt with ablation energy.

### DETAILED DESCRIPTION

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

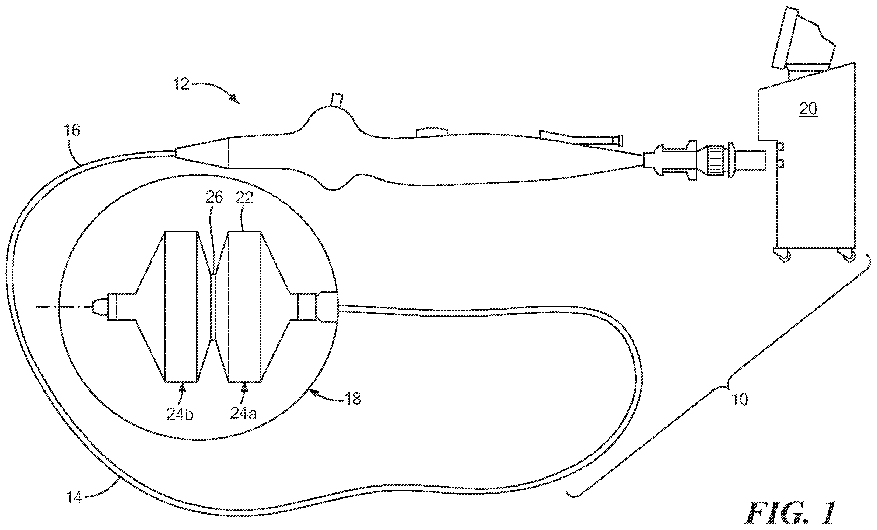

Referring now to FIG. 1 in which an exemplary system and medical device is shown for creating and atrial shunt and designated generally as "10." The system 10 may include a medical device 12 having an elongate body 14 defining a major longitudinal axis and having a proximal portion 16 and a distal portion 18. The proximal portion 16 of the medical device 12 is configured to couple with a controller or console 20 configured to deliver ablation energy to the distal portion 18 of the medical device 12. In one configuration, the controller 20 is a cryogenic console having a cryogenic fluid source in fluid communication with the console and a scavenging line in communication with the medical device 12 to recycle cryogenic fluid used during treatment. The distal portion 18 of the medical device 12 includes a balloon 22, which may be adjustable and/or manipulatable. The balloon 22 is sized and configured to expand to ablate a septal wall of a mammalian heart. The balloon 22 may be compliant or non-compliant. In one configuration, the balloon 22 includes a pair of longitudinally spaced lobes 24a and 24b. The lobes 24a and 24b may be substantially the same size and a shape, each lobe having a first diameter when inflated that is substantially the same. A middle portion 26 having a second diameter less than the first diameter is disposed between the first lobe 24a and the second lobe 24b. In the configuration shown in FIG. 1, the middle portion 26 further defines a length less than a length of each of the first lobe 24a and the second lobe 24b. In another configuration, the diameter of each lobe 24a and 24b may be adjustable and may be different. For example, the first lobe 24a may be larger in diameter than the second lobe 24b or vice versa.

Referring now to FIGS. 1-3, the first lobe 24a and the second lobe 24b are sized and configured to, when inflated, abut and thermally isolate an atrial septum from blood flowing within a left atrium and a right atrium when the balloon is disposed within an atrial shunt. In particular, as shown in FIG. 1, each lobe 24a and 24b tapers is diameter as they extend toward the middle portion 26. This tapering creates a wedge shape as between the first lobe 24a, the second lobe 24b, and the middle portion 26. This wedge shape traps the septal wall being ablated from both sides of the septal wall and thereby isolating blood that is flowing within respective atria from the septal wall being ablated. This prevents a warming effect from the blood on the septal wall, which would prevent the septal wall tissue from being ablated and allow to freeze over a larger band. Moreover, by having the tissue held on both side over a larger area, this reduce the risk of tearing down the tissues and ripping it as the forces applied on the device to procced are distributed over a larger surface of the septum and not only around its circumference.

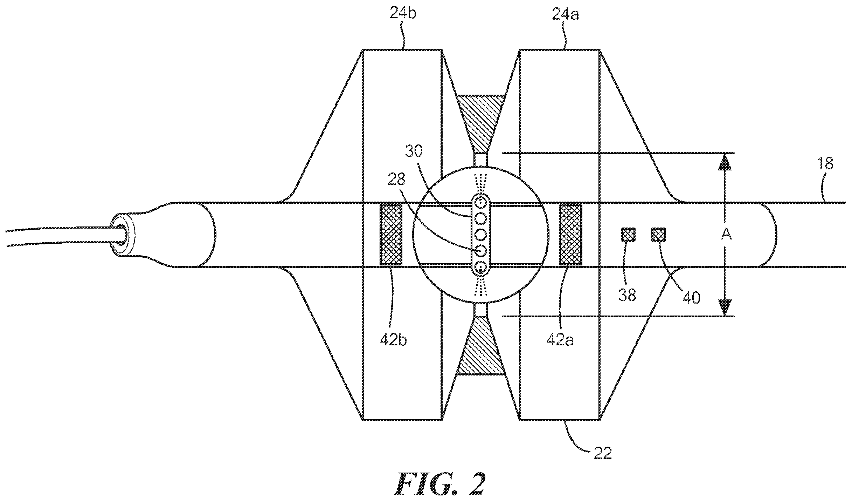

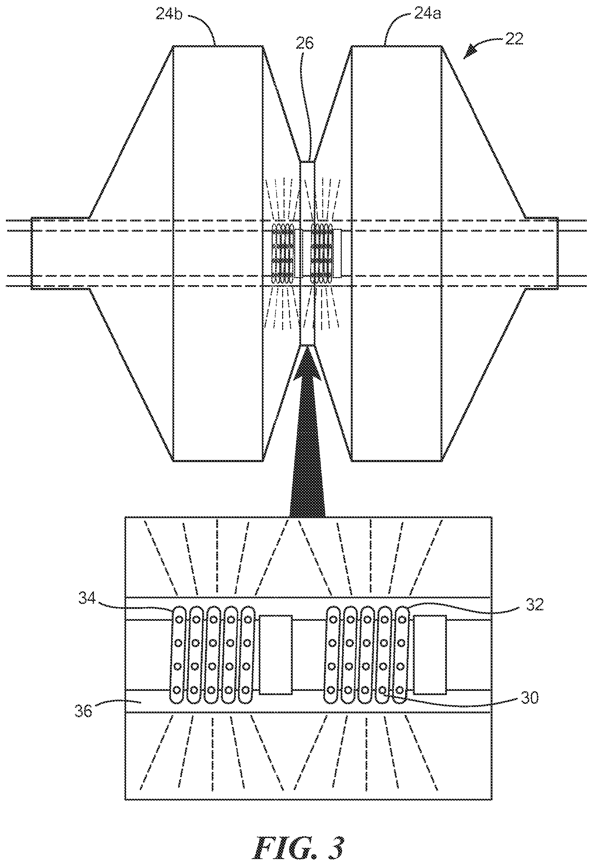

To further isolate the septal wall for treatment, an ablation element 28 is disposed substantially within the middle portion 26. The ablation element 28 is configured to deliver cryogenic ablation energy, such as a dose of refrigerant or coolant, solely to the middle portion 26 to avoid collateral damage to surrounding tissue or blood other than the septal wall. When refrigerant (i.e., cryogenic ablation energy) is delivered to the middle portion 26, the inner and/or outer surface of the middle portion 26 is cooled to a temperature sufficient for extracting heat energy from, and therefore ablating, target tissue. In one configuration, the ablation element 28 includes a first plurality of spray ports 30 circumferentially disposed about the elongate body 14 within the middle portion 26 and configured to deliver refrigerant to the middle portion 26. In the configuration shown in FIG. 2, each of the first plurality of spray ports 30 is angled in a direction orthogonal to the major longitudinal axis and directed at the portion of the atrial wall wedged between the first lobe 24a and the second lobe 24b. The first plurality of spray ports 30 may be disposed along a first coiled fluid injection tube 32 (shown FIG. 3) that extends along and wraps about the elongate body 14. In one configuration, as shown in FIG. 3, a second plurality of spray ports 34 is included within the middle portion 26 and longitudinally spaced from the first plurality of spray ports 30. The second plurality of spray ports 34 may be included on a second coiled fluid injection tube 36 that extends along and wraps about the elongate body 14. As shown in FIG. 3, in such a configuration with two pluralities of spray ports 30 and 34 circumferentially disposed about the elongate body 14 in the middle portion 26, the spray ports 30 and 34 may be angled to direct cryogenic fluid directly toward the septal tissue being ablated. A temperature sensor 38 and a pressure sensor 40 may further be included in one of the first lobe 24A and the second lobe 24B to monitor the temperature and pressure of the balloon 22 during an ablation procedure. Moreover, a pair of radiopaque markers 42A and 42B may be included on opposite sides of the middle portion 26 to aide in the proper alignment of the balloon 22 within the atrial shunt under fluoroscopy.

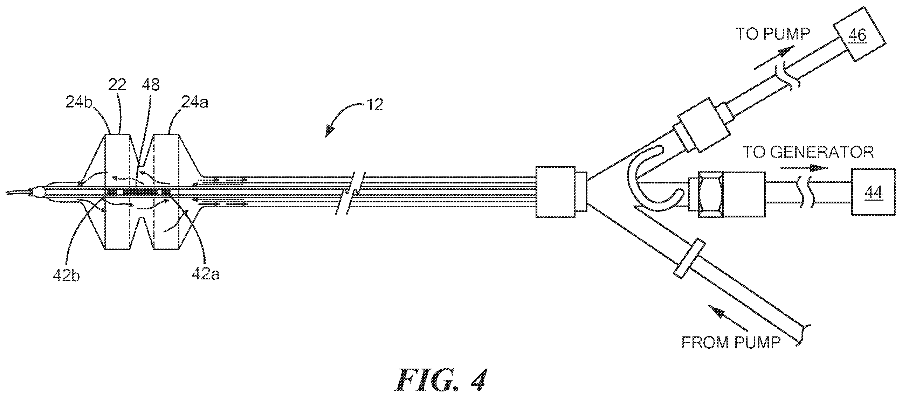

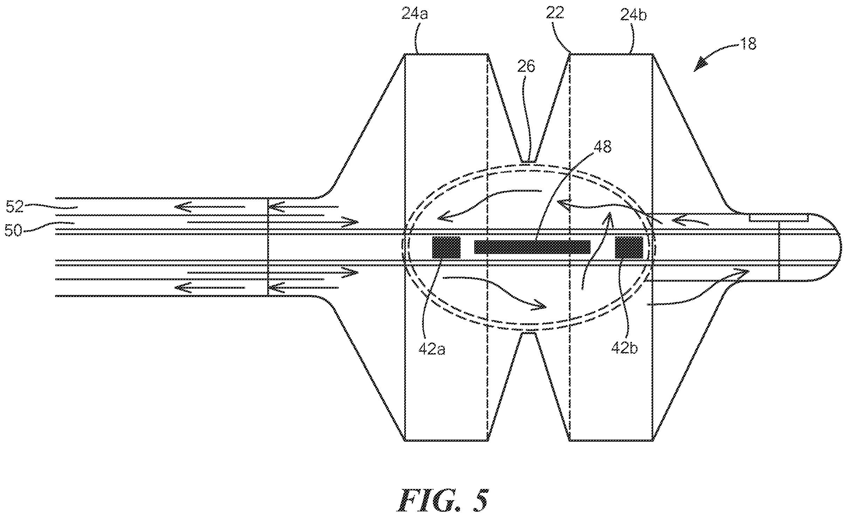

Referring now to FIGS. 4-5, in another configuration, the medical device 12 may be configured to deliver radiofrequency energy, such as microwave energy, to atrial septum to create an atrial shunt. The medical device 12 may be in communication with a radiofrequency (RF) generator 44 and a fluid supply 46, such a saline, configured to inflate the balloon 22. In the configuration shown in FIG. 5, an elongated radiator 48 is substantially disposed within the middle portion 26 of the balloon 22 and disposed between the radiopaque markers 42a and 42b and configured to deliver RF ablation energy to the septal wall. The radiator 48 may include various radiating structures, for example, monopole, dipole, folded dipole, coil, and a co-axial slot. The radiator 48 may include structural elements with conductive and non-conductive materials to shape the radiating field generated by the radiator, such as dielectric loading elements and phase cancelling structures. In such a configuration, the medical device 12 may include a fluid delivery tube 50 configured to deliver fluid to inflate the balloon 22 and an exhaust tube 52 configured to exhaust saline from the balloon 22 to deflate it.

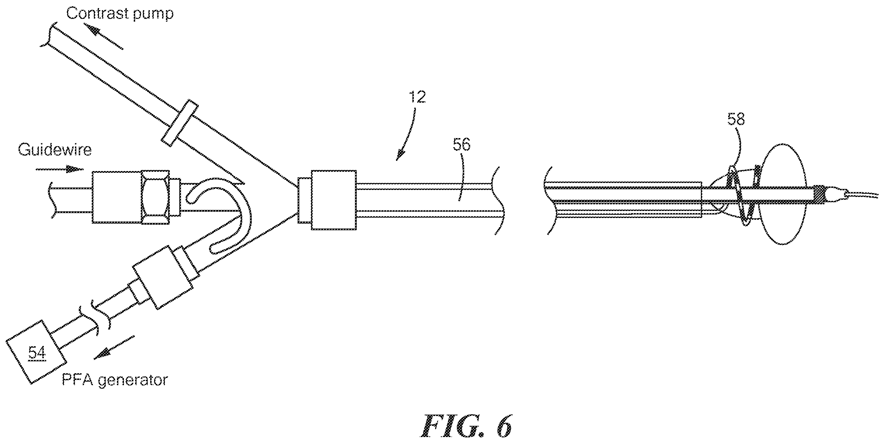

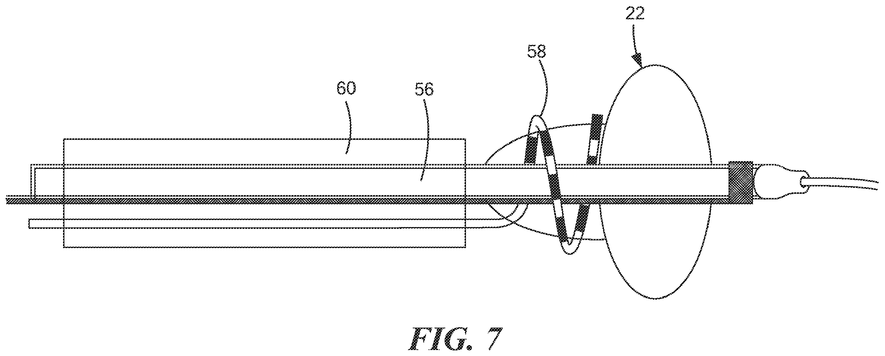

Referring now to FIGS. 6-7, in another configuration, the medical device 12 may be configured to deliver pulse field ablation to the septal wall to cause electroporation of the target tissue. The medical device 12 may be in communication with a pulse field ablation generator 54 configured to deliver high voltage pulses of energy to the target tissue. In one configuration, a catheter 56 having a plurality of electrodes 58 is configured to be advanced alongside the elongate body 14 within an outer catheter 60 disposed around the catheter 56. The plurality of electrodes 58 are configured to be manipulated to define a circumferential and planar configuration proximal to the balloon 22.

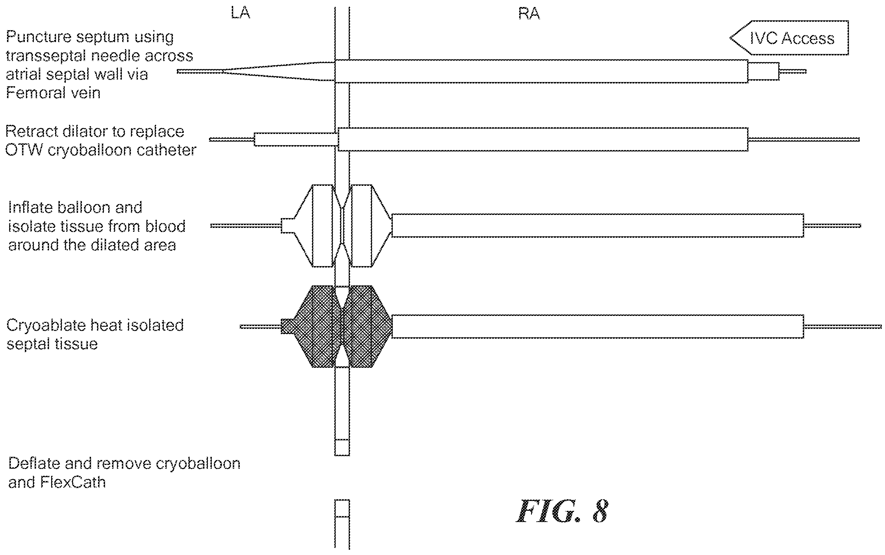

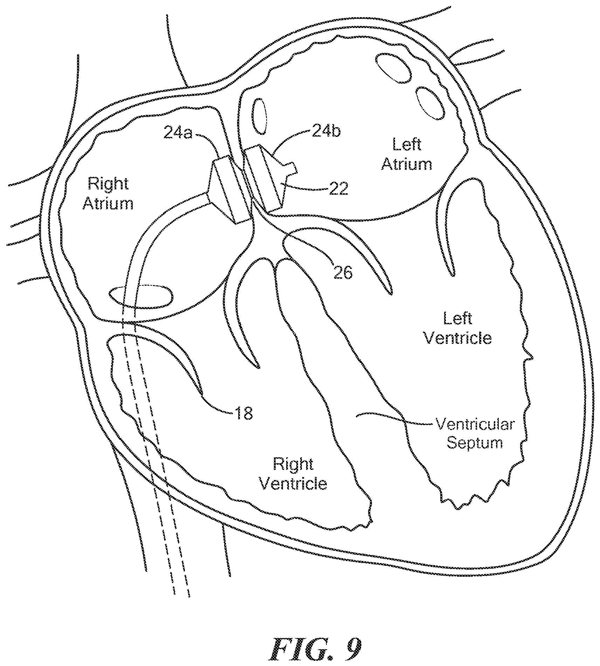

Referring now to FIG. 8, a method of creating an atrial shunt includes puncturing the atrial septum between the right atrium and the left atrium to create a shunt. For, example, a transseptal needle may be advanced through the femoral vein and across the septal wall to create an opening or shunt. An ablation device 12 having the balloon 22 is advanced at least partially through the opening. For example, the transseptal puncturing device may have partially opened the opening with a dilator and the ablation device 12 may be advanced over a guidewire for placement of the balloon 22 within the opening. The balloon 22 may either be inflated before or during ablation begins. For example, during an RF ablation procedure the balloon 22 may be inflated with saline before RF energy is delivered, or may be inflated with refrigerant, from the first plurality of spray ports 30 and/or second plurality of spray ports 34 during the ablation procedure. In one configuration, the balloon is inflated to 8atm. As discussed above, the balloon 22 is configured to thermally isolate the atrial septum from blood within the left atrium and the right atrium. In another words, the middle portion 26 of balloon 22 is advanced to a position where it is aligned with the atrial septum and the first lobe 24a abuts one side of the septal wall and the second lobe 24b abuts the opposite side of the septal wall, as shown in FIG. 9. Refrigerant is delivered to the balloon by spraying refrigerant to the middle portion 24 of the balloon 22 to ablate the septal wall and create the shunt. Once the ablation procedure is completed, the balloon 22 may be retracted and the shunt remains open without any additional mechanical device being inserted within the shunt to maintain the opening.

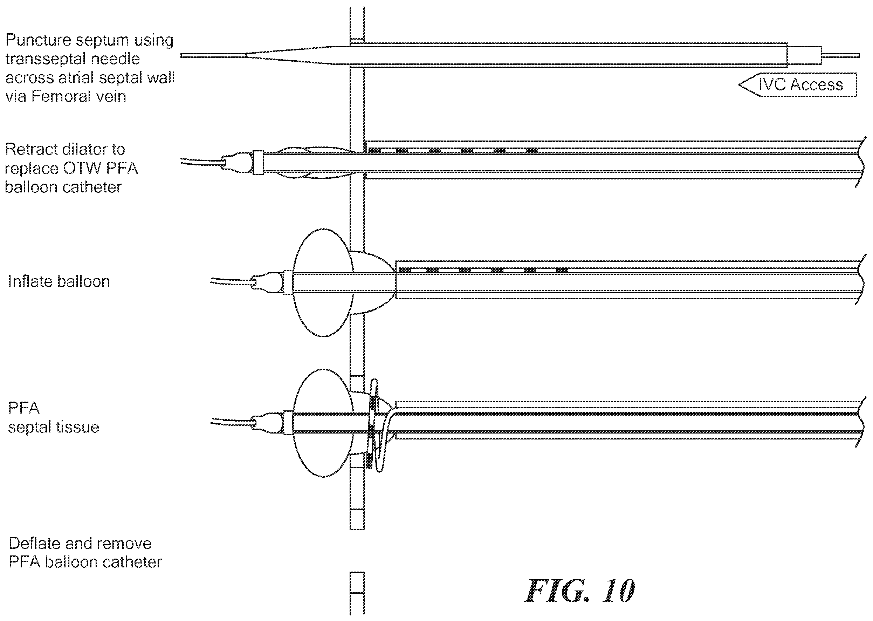

Referring now to FIG. 10, in another method of creating an atrial shunt, pulse field ablation energy is used to create the atrial shunt and without the need for a mechanical device within the shunt following the procedure. For, example, a transseptal needle may be advanced through the femoral vein and across the septal wall to create an opening or shunt. An ablation device 12 having the balloon 22 is advanced at least partially through the opening to a first side of the atrial wall. For example, the transseptal puncturing device may have partially opened the opening with a dilator and the ablation device 12 may be advanced over a guidewire for placement of the balloon 22 at least partially within the opening. The balloon 22 may be inflated with saline before pulse field ablation energy is delivered from the first plurality of spray ports 30 and/or second plurality of spray ports 34 during the ablation procedure. In one configuration, the balloon is inflated to 8atm and is a single lobed balloon or a dual lobed balloon, whether compliant or non-compliant. The balloon 22 is positioned so that it abuts the septal wall from the opposite side of the septal wall from the plurality of electrodes 58. The balloon 22 holds open a portion the septal wall while the plurality of electrodes 58 ablate the septal wall around the balloon 22. Once the septal wall is ablated, the ablation device 12 is retracted as well as the plurality of electrodes 58 and the created shunt remains open without a mechanical device.

In another configuration, a first of the plurality of electrodes 58 is included on the middle portion 26 of the balloon 22 in a catheter 56 with a second and third of the plurality of electrodes 58 positioned on opposite sides of the balloon lobes 24a and 24a. Pulse field ablation energy may be delivered in a bipolar manner as between the first of the plurality of electrodes 58 and at least one of the second and third of the plurality of electrodes 58 to non-thermally ablated opposite side of the septal wall. In another configuration, the catheter 56 include a single lobed balloon 22 which is pulled or pushed against the septal puncture, which a catheter 56 mounted electrode 58 in contact with one side of the septum. An electrical return path electrode is positioned on the catheter 56 on the other side of the septum to which pulse field ablation energy is delivered from the electrode 58 in contact with the other side of the septum. In still other configurations, no balloon 22 is included and a pair of electrodes on either side of the septum are used to ablate the septal wall with bipolar pulse field ablation energy.

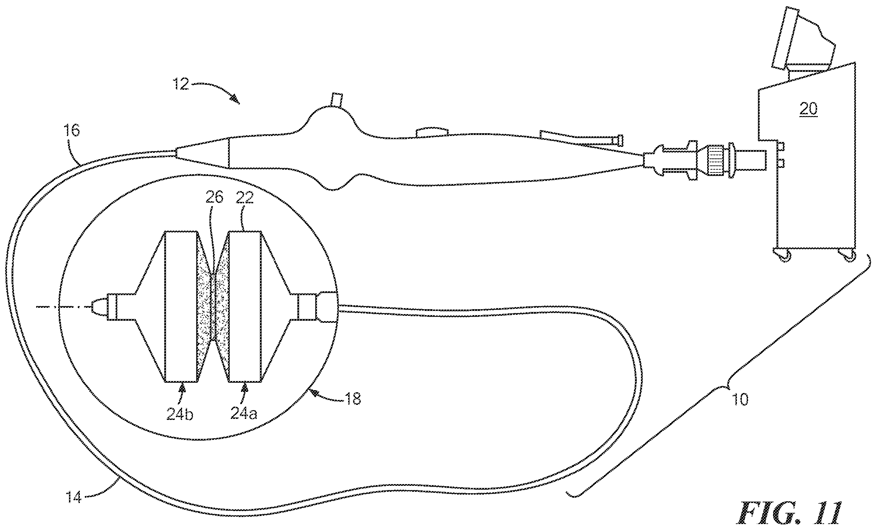

Referring now to FIGS. 11-13, the balloon 22 may be coated with and/or elute a therapeutic drug or chemical that inhibits the closure of the shunt following an ablation and/or electroporation procedure. For example, in one embodiment, the balloon 22 may be coated with and/or elute at least one therapeutic drug or chemical such as alcohol, sclerosing agents, cryogenic adjuvants, electroporation adjuvants, and calcium phosphate. The drug may be coated onto an exterior surface of the balloon 22 such that the coating may contact the exposed and/or dilated septal tissue when the balloon 22 is inflated within the shunt prior to ablation of the tissue. The therapeutic drug may be coated onto the exterior surface of the balloon 22 using various techniques such as, for example, spray-coating, dip-coating, brushing, roll-coating, etc. In one embodiment, an outer surface of each lobe 24a and 24b, and the middle portion 26 of the balloon 22 may be coated with or elute the drug. However, it is to be understood that the balloon 22 may be coated such that only a portion of the balloon 22 may be coated with the drug. For example, in one embodiment, only one of the lobes 24a or 24b, or only the middle portion 26, may be coated with the drug.

Treating the tissue with the drug prior to the ablation procedure may improve the effect of the ablation by reducing thrombosis, restenosis, and favor calcification of the septum area surrounding the shunt to create a calcified ring that mechanically holds the interatrial shunt open and prevents tissue regrowth. In other words, the drug may create a ring of fibrosis or calcifications to ensure that the shunt is mechanically stable with a predefined diameter (such as, for example, 8 mm). The drug eluting or coated balloon 22 may be used in either a cryoablation procedure, radiofrequency (RF) ablation, or an electroporation procedure. When used in an electroporation procedure, the delivery of pulsed field ablation energy may enable deeper penetration of the therapeutic drug within the target tissue cells, thereby increasing the effectiveness of the procedure. Additionally, in RF ablation and electroporation procedures, an expandable element or stent may also be used and coated with or elute the therapeutic drug. When used during a cryoablation procedure, the drug may help attain lower tissue treatment temperatures and/or affect microcirculation, which in turn could create deeper, larger, or more damaging lesions that may be necessary for permanent inter atrial shunt formation, and precipitate a mechanically stable ring of tissue fibrosis or calcifications. Because cell porosity may be affected by the freeze-thaw-freeze cycles of cryoablation procedures, this phenomenon can be leveraged to enable penetration of the drug at the lesion periphery where cryoablation effectiveness is not as critical.

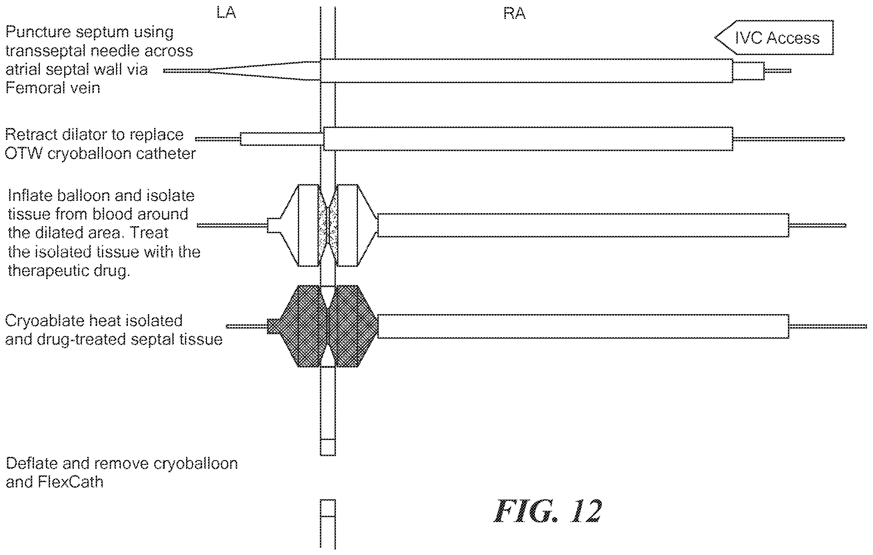

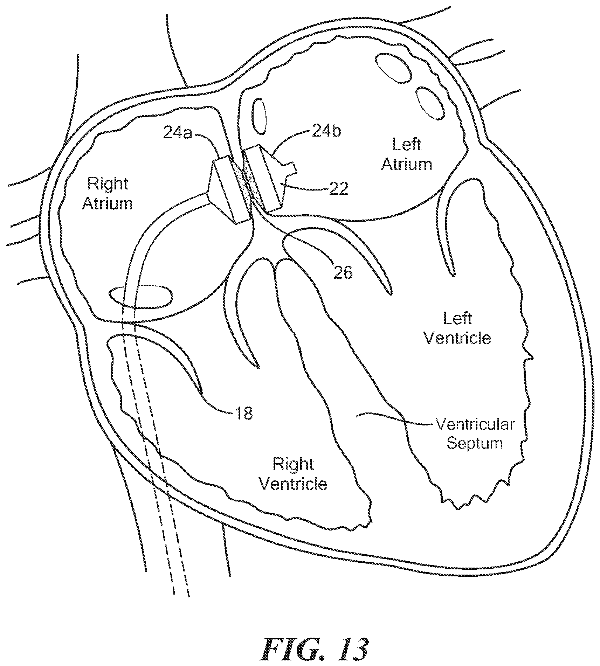

Now referring to FIG. 12, in which a step-by-step method of creating an interatrial shunt is shown. First, a transseptal needle may be advanced through the femoral vein and across the septal wall to create an opening between the right atrium and the left atrium. The ablation device 12 having the balloon 22 is then advanced at least partially through the opening to a first side of the atrial wall. Once the ablation device 12 has been advanced at least partially through the opening, the balloon 22 is then inflated and thermally isolates the atrial septum from blood within, or passing between, the left atrium and right atrium (as shown in FIG. 13). During the inflation of the balloon 22, a therapeutic drug that is eluting from or coated on an exterior surface of the balloon 22 comes into contact with the shunt tissue, thereby treating the tissue and inhibiting tissue regrowth so that the opening remains open following the ablation treatment. In other words, the therapeutic drug may be used to slow or inhibit the regrowth of tissue following the ablation treatment which forms the interatrial shunt. The therapeutic drug may be at least one chemical or mixture of chemicals selected from the group consisting of alcohol, sclerosing agents, cryogenic adjuvants, and calcium phosphate. Following the treatment of the shunt with the therapeutic drug, a dose of refrigerant may then be sprayed towards the middle portion 24 of the balloon 22 from the first and/or second plurality of spray ports 30, 34 to cool the balloon 22 to a temperature sufficient for extracting heat energy from the septal wall, thus ablating the tissue defining the opening and creating the shunt. Once the ablation procedure is completed (the shunt is formed), the balloon 22 may be retracted and the shunt remains open without any additional mechanical device being inserted within the shunt to maintain the opening. It will be appreciated by persons skilled in the art that the present invention is not limited to what has been particularly shown and described herein above. In addition, unless mention was made above to the contrary, it should be noted that all of the accompanying drawings are not to scale. A variety of modifications and variations which fall under the scope of the claims are possible

## Claims

1. A medical device comprising:
an elongate body (14) defining a major longitudinal axis and having a proximal portion (16) and a distal portion (18);
a balloon (22) coupled to the distal portion;
a therapeutic drug coated around an outer surface of the balloon, the therapeutic drug being a chemical used to inhibit tissue growth; and
an ablation element (28) disposed substantially within the balloon and configured to deliver ablation energy to an interior surface of the balloon.

2. The device of claim 1, wherein the ablation energy is a dose of refrigerant.

3. The device of claim 2, wherein the ablation element includes a first plurality of spray ports (30) configured to deliver the dose of refrigerant to the interior surface of the balloon.

4. The device of claim 3, wherein the first plurality of spray ports is angled in a direction substantially orthogonal to the major longitudinal axis.

5. The device of claim 2 or 3, wherein the balloon includes a pair of longitudinally spaced lobes (24a, 24b) having a first diameter and a middle portion (26) having a second diameter less than the first diameter disposed therebetween, the device further including a second plurality of spray ports within the middle portion and longitudinal spaced from the first plurality of spray ports.

6. The device of any one or more of claims 1-4, wherein the balloon includes a pair of longitudinally spaced lobes (24a, 24b) having a first diameter and a middle portion (26) having a second diameter less than the first diameter disposed therebetween.

7. The device of claim 6, wherein the pair of longitudinally spaced lobes are sized and configured to, when inflated, abut and thermally isolate an atrial septum from blood flowing within a left atrium and a right atrium when the balloon is disposed within an atrial shunt.

8. The device of any one or more of claims 1-7, wherein the ablation element is configured to deliver a dose of refrigerant to the interior surface of the balloon.

9. The device of claim 8, wherein the dose of refrigerant is configured to cool the balloon to a temperature sufficient to extract heat from an area of tissue within an atrial septum.

10. The device of claim 8 or 9, wherein the balloon includes a pair of longitudinally spaced lobes (24a, 24b) having a first diameter and a middle portion (26) having a second diameter less than the first diameter disposed therebetween, wherein the ablation element is configured to spray the dose of refrigerant to the middle portion of the balloon.

11. The device of any one or more of claims 1-10, wherein the therapeutic drug is at least one chemical selected from the group consisting of alcohol, sclerosing agents, cryogenic adjuvants, and calcium phosphate.

## Patentansprüche

1. Medizinische Vorrichtung, umfassend:
einen länglichen Körper (14), der eine Hauptlängsachse definiert und einen proximalen Abschnitt (16) und einen distalen Abschnitt (18) aufweist,
einen an den distalen Abschnitt gekoppelten Ballon (22),
ein therapeutisches Medikament, mit dem eine Außenfläche des Ballons beschichtet ist, wobei das therapeutische Medikament eine zum Hemmen von Gewebewachstum verwendete Chemikalie ist, und
ein Ablationselement (28), das im Wesentlichen in dem Ballon angeordnet und dazu ausgestaltet ist, Ablationsenergie an eine Innenfläche des Ballons abzugeben.

2. Vorrichtung nach Anspruch 1, wobei die Ablationsenergie eine Kältemitteldosis ist.

3. Vorrichtung nach Anspruch 2, wobei das Ablationselement eine erste Vielzahl von Sprühöffnungen (30) aufweist, die dazu ausgestaltet sind, die Kältemitteldosis an die Innenfläche des Ballons abzugeben.

4. Vorrichtung nach Anspruch 3, wobei die erste Vielzahl von Sprühöffnungen in einer im Wesentlichen orthogonal zu der Hauptlängsachse verlaufenden Richtung abgewinkelt sind.

5. Vorrichtung nach Anspruch 2 oder 3, wobei der Ballon ein Paar in Längsrichtung beabstandeter Lappen (24a, 24b) mit einem ersten Durchmesser und einen dazwischen angeordneten mittleren Abschnitt (26) mit einem zweiten Durchmesser, der kleiner als der erste Durchmesser ist, aufweist, wobei die Vorrichtung ferner eine von der ersten Vielzahl von Sprühöffnungen in Längsrichtung beabstandete zweite Vielzahl von Sprühöffnungen innerhalb des mittleren Abschnitts aufweist.

6. Vorrichtung nach einem oder mehreren der Ansprüche 1 - 4, wobei der Ballon ein Paar in Längsrichtung beabstandeter Lappen (24a, 24b) mit einem ersten Durchmesser und einen dazwischen angeordneten mittleren Abschnitt (26) mit einem zweiten Durchmesser, der kleiner als der erste Durchmesser ist, aufweist.

7. Vorrichtung nach Anspruch 6, wobei das Paar in Längsrichtung beabstandeter Lappen so bemessen und ausgestaltet sind, dass sie, wenn sie inflatiert sind, an einem Vorhofseptum anliegen und dieses thermisch gegenüber Blut isolieren, das in einem linken Vorhof und einem rechten Vorhof fließt, wenn der Ballon in einem Vorhofshunt angeordnet ist.

8. Vorrichtung nach einem oder mehreren der Ansprüche 1 - 7, wobei das Ablationselement dazu ausgestaltet ist, eine Kältemitteldosis an die Innenfläche des Ballons abzugeben.

9. Vorrichtung nach Anspruch 8, wobei die Kältemitteldosis dazu ausgestaltet ist, den Ballon auf eine Temperatur abzukühlen, die zum Abziehen von Wärme aus einem Gewebebereich innerhalb eines Vorhofseptums ausreicht.

10. Vorrichtung nach Anspruch 8 oder 9, wobei der Ballon ein Paar in Längsrichtung beabstandeter Lappen (24a, 24b) mit einem ersten Durchmesser und einen dazwischen angeordneten mittleren Abschnitt (26) mit einem zweiten Durchmesser, der kleiner als der erste Durchmesser ist, aufweist, wobei das Ablationselement zum Sprühen der Kältemitteldosis auf den mittleren Abschnitt des Ballons ausgestaltet ist.

11. Vorrichtung nach einem oder mehreren der Ansprüche 1 - 10, wobei das therapeutische Medikament mindestens eine aus der aus Alkohol, Sklerosierungsmitteln, kryogenen Hilfsstoffen und Calciumphosphat bestehenden Gruppe ausgewählte Chemikalie ist.

## Revendications

1. Dispositif médical comprenant :
un corps allongé (14) définissant un axe longitudinal principal et possédant une partie proximale (16) et une partie distale (18) ;
un ballonnet (22) couplé à la partie distale ;
un médicament thérapeutique revêtu autour d'une surface externe du ballonnet, le médicament thérapeutique étant un produit chimique utilisé pour inhiber la croissance tissulaire ; et
un élément d'ablation (28) disposé sensiblement à l'intérieur du ballonnet et configuré pour fournir de l'énergie d'ablation à une surface intérieure du ballonnet.

2. Dispositif selon la revendication 1, l'énergie d'ablation étant une dose de réfrigérant.

3. Dispositif selon la revendication 2, l'élément d'ablation comprenant une première pluralité d'orifices de pulvérisation (30) configurés pour délivrer la dose de réfrigérant à la surface intérieure du ballonnet.

4. Dispositif selon la revendication 3, la première pluralité d'orifices de pulvérisation étant inclinée dans une direction sensiblement orthogonale au grand axe longitudinal.

5. Dispositif selon la revendication 2 ou 3, le ballonnet comprenant une paire de lobes espacés longitudinalement (24a, 24b) ayant un premier diamètre et une partie médiane (26) ayant un second diamètre inférieur au premier diamètre disposée entre eux, le dispositif comprenant en outre une seconde pluralité d'orifices de pulvérisation à l'intérieur de la partie médiane et espacés longitudinalement de la première pluralité d'orifices de pulvérisation.

6. Dispositif selon l'une quelconque ou plusieurs des revendications 1 à 4, le ballonnet comprenant une paire de lobes espacés longitudinalement (24a, 24b) ayant un premier diamètre et une partie médiane (26) ayant un second diamètre inférieur au premier diamètre disposée entre eux.

7. Dispositif selon la revendication 6, la paire de lobes espacés longitudinalement étant dimensionnés et configurés pour, lorsqu'ils sont gonflés, buter contre et isoler thermiquement un septum auriculaire du sang circulant dans une oreillette gauche et une oreillette droite lorsque le ballonnet est disposé dans un shunt auriculaire.

8. Dispositif selon l'une quelconque ou plusieurs des revendications 1 à 7, l'élément d'ablation étant configuré pour délivrer une dose de réfrigérant à la surface intérieure du ballonnet.

9. Dispositif selon la revendication 8, la dose de réfrigérant étant configurée pour refroidir le ballonnet à une température suffisante pour extraire la chaleur d'une zone de tissu à l'intérieur d'un septum auriculaire.

10. Dispositif selon la revendication 8 ou 9, le ballonnet comprenant une paire de lobes espacés longitudinalement (24a, 24b) ayant un premier diamètre et une partie médiane (26) ayant un second diamètre inférieur au premier diamètre disposé entre eux, l'élément d'ablation étant configuré pour pulvériser la dose de réfrigérant sur la partie médiane du ballonnet.

11. Dispositif selon l'une quelconque ou plusieurs des revendications 1 à 10, le médicament thérapeutique étant au moins un produit chimique choisi dans le groupe constitué par l'alcool, les agents sclérosants, les adjuvants cryogéniques et le phosphate de calcium.
